# FASCICULE DE BREVET EUROPEEN

(11) **EP 0 971 702 B1**
(45) Date de publication et mention de la délivrance du brevet: **03.12.2003**
(21) Numéro de dépôt: 98908157.5
(22) Date de dépôt: 10.02.1998
(51) Int. Cl.: A61K 9/16, A61K 9/50

(54) **MICROGRANULES CONTENANT DU CISPLATINE**
CISPLATINHALTIGE MIKROGRANULATE
MICROGRANULES CONTAINING CISPLATIN

(30) Priorité: 11.02.1997 FR 9701545
(43) Date de publication de la demande: 19.01.2000
(73) Titulaire: Laboratoires des Produits Ethiques Ethypharm, 78550 Houdan (FR)
(72) Inventeur: DEBREGEAS, Patrice, F-75007 Paris (FR); LEDUC, Gérard, F-45330 Malesherbes (FR); OURY, Pascal, F-75005 Paris (FR); SUPLIE, Pascal, F-27400 Montaure (FR)
(74) Mandataire: Warcoin, Jacques
(86) Numéro de dépôt international: FR9800251
(87) Numéro de publication internationale: WO98034599

(56) Documents cités:
- EP-A- 0 167 825
- WO-A-96/27346
- DE-A- 3 523 454
- DATABASE WPI Week 9619 Derwent Publications Ltd., London, GB; AN 96-184677 [19] XP002044628 & JP 08 059 460 A (TANAKA KIKINZOKU KOGYO KK) 5 mars 1996
- DATABASE WPI Week 9349 Derwent Publications Ltd., London, GB; AN 93-392570 [49] XP002044629 & JP 05 294 839 A (BIOMATERIAL UNIVERSE KK) 9 novembre 1993

## Description

La présente invention concerne une formulation du cisplatine pour administration orale.

Le cisplatine est un agent anticancéreux connu pour son efficacité mais aussi pour ses effets secondaires notables, observés lorsqu'il est administré par voie intraveineuse, notamment : néphrotoxicité, toxicité gastrointestinale (nausées, vomissements), neurotoxicité et myélosuppression modérée.

La néphrotoxicité induite par le cisplatine peut être atténuée par hydratation saline intraveineuse et par diurèse.

Depuis ces vingt-cinq dernières années, des recherches ont été menées sur des analogues du cisplatine. Seulement douze de ces analogues ont été évalués au cours d'essais cliniques : certains se sont avérés encore plus toxiques que le cisplatine et aucun n'a montré une activité anticancéreuse supérieure au cisplatine.

Les chercheurs se sont donc tournés vers l'étude de la réduction de toxicité du cisplatine plutôt que vers celle de nouveaux analogues.

Un premier axe de recherche concerne l'administration orale du cisplatine chez les animaux.

Les études réalisées par Siddik Z.H. et al. et présentées au 74ème congrès de l'Association Américaine de Recherche contre le Cancer en mars 1984 ont permis d'évaluer l'activité anticancéreuse du cisplatine administré par voie orale chez des souris atteintes de plasmacytome ADJ/PLA. La concentration plasmatique en platine a atteint un pic de 4,3 µg/ml au bout de 30-60 minutes pour une dose de 50 mg/kg. La biodisponibilité chez la souris était de 31-36 % et l'incidence de la néphrotoxicité était seulement de 20 %.

Hasegawa Y. et al. confirment sur des muridés, dans Chem. Pharm. Bull. 33(12), 5511-5514, 1985, que le cisplatine passe dans le sang après administration orale et qu'il est efficace dans ces conditions contre les tumeurs solides.

Binks S.P. et al. démontrent dans Biochemical Society Transactions, 616th Meeting, London, 14, 694 (1986), que l'absorption du cisplatine après administration orale est tellement rapide que la concentration plasmatique en platine atteint son maximum en moins de deux heures. Les taux les plus élevés de platine sont observés dans les reins.

L'analyse au microscope électronique des tissus excisés 48 heures après l'administration orale du cisplatine ne révèle que peu de changements dans les reins, tandis que pour une administration intraveineuse, les symptômes de néphrotoxicité sont observables.

Borch R.F. et al. ont montré dans Proc. Natl. Acad. Sci., USA, 76, 6611-6614, que les concentrations d'urée dans le sang ont été multipliées par 14 chez des rats recevant la dose maximale tolérée de cisplatine par voie intraveineuse puis ce résultat a été confirmé par une autre étude de Morgan S.E. et al. (Pharmacology Communication, 1993, vol. 3, n° 1, 9-18) et montre que l'administration par voie orale peut diminuer largement la néphrotoxicité du cisplatine. Aucun changement histopathologique au niveau des reins n'a été observé chez la souris traitée oralement par une dose toxique de cisplatine de 70 mg/kg.

Howell S.B. dans Plenum Press. New York, p. 93 (1991) et Harrap K.R. et al. dans Adv. Enzyme Regul. 31 (31), 1991, révèlent que le degré d'activité du cisplatine administré par voie orale est moindre que celui obtenu par voie parentérale et que des doses plus élevées sont nécessaires pour une administration orale en raison de la biodisponibilité relativement faible du cisplatine dans ce cas. En conséquence, aucun essai clinique n'a été conduit sur l'homme parce que la biodisponibilité du cisplatine administré par voie orale était trop faible comparé aux formulations intraveineuses classiques.

Un deuxième axe de recherche concerne l'association de faibles doses de cisplatine avec d'autres modes de thérapie.

T. Shirasaka a proposé dans Cancer Chemother. Pharmacol., 32, 167-172 (1993), et dans Jpn. J. Cancer Chemother. 2/(7) 1025-1028 (1994), une thérapie qui consiste à combiner l'administration de 5-fluoro-uracil et du cisplatine à faible dose. Il suggère une thérapie de quatre semaines, qui consiste à administrer une perfusion de 5-fluoro-uracil associée à une dose intraveineuse de 5-6 mg/jour de cisplatine.

L'efficacité anticancéreuse de cette thérapie sur les tumeurs solides des rongeurs étudiés est supérieure à celle du 5-fluoro-uracil seul ou du cisplatine seul et sa toxicité est moindre.

De nombreuses études effectuées au Japon ont montré qu'avec un régime à faible dose, le cisplatine associé à du 5-fluoro-uracil est efficace dans le traitement de divers cancers, de surcroît sa toxicité est amoindrie. Il n'est alors plus nécessaire d'avoir recours à une hydratation par voie intraveineuse pour prévenir la néphrotoxicité.

Il a, par ailleurs, été démontré dans Chemotherapy, 1996, Vol/Iss/Pg 42/6 (452-458) que le cisplatine à faible dose peut aussi être associée à du S1, médicament antitumoral de forme orale qui est un mélange tegafur/5-chloro-2,4-dihydroxypyridine/acide oxonique dans une proportion molaire 1/0,4/1, découvert par T. Shirasaka.

D'autres associations du cisplatine à faible dose sont aussi possibles avec d'autres anticancéreux tels que, mais non limités, à l'association de la vinblastine avec la bléomycine, l'association de l'étoposide avec la bléomycine, ou encore du paclitaxel.

En outre, Ducreux M. et al. dans Annals of Oncology, 5 (Suppl. 8), 81 (1994), ont montré que l'association de la radiothérapie et d'une administration de 4-6 mg/m²/jour de cisplatine par voie intraveineuse pendant 4-6 semaines améliorait l'activité anticancéreuse et réduisait les effets secondaires.

Il n'existe actuellement aucune formulation orale du cisplatine et l'objet de la présente invention est de fournir des microgranules à libération contrôlée, pour administration orale, contenant du cisplatine, dont la granulométrie moyenne est comprise entre 0,4 et 1,5 mm, notamment entre 1 et 1,25 mm.

On entend par libération contrôlée, une libération instantanée, prolongée dans le temps ou encore avec ciblage du site d'absorption, notamment au niveau de l'iléon où le pH est de l'ordre de 7.

Cette formulation fournit avantageusement une biodisponibilité supérieure à celle du cisplatine de forme injectable administrée oralement et une toxicité gastro-intestinale acceptable.

Après une administration intraveineuse, la concentration plasmatique en platine augmente puis diminue rapidement, ce qui engendre des fluctuations de concentration importantes et crée des périodes de sous-et de surconcentration thérapeutique, responsables de la néphrotoxicité et des nausées-vomissements.

La formulation microgranules à libération contrôlée selon l'invention permet avantageusement de libérer le principe actif plus régulièrement et d'éviter des pics plasmatiques en maintenant un taux sanguin suffisamment élevé pour obtenir l'effet thérapeutique voulu, sans toutefois atteindre des niveaux toxiques pouvant entraîner des effets secondaires pour le patient, en raison de la large répartition des granules le long du tube digestif.

La formulation selon l'invention permet également de maintenir la concentration plasmatique constante sur une période de temps plus longue, de diminuer les variations inter- et intra-individuelles grâce à une surface d'échange élevée et évite la libération d'une quantité importante de principe actif localisée en un point de la muqueuse digestive.

Un avantage de la forme orale selon l'invention est d'être utilisable par le patient lui-même à son domicile ; ainsi le patient n'a plus à recourir à des administrations intraveineuses fréquentes à l'hôpital qui nécessitent une assistance professionnelle. De plus, pour les patients hospitalisés, la forme orale selon l'invention améliore la qualité de vie en réduisant le temps passé à l'hôpital et en les affranchissant de traitements pénibles, en particulier dans le cas de perfusions à raison de 100 heures/semaine.

Chaque microgranule selon l'invention comprend avantageusement un microgranule immédiat sur lequel est monté un revêtement contenant un agent d'enrobage permettant la libération contrôlée du cisplatine et/ou d'autres principes actifs, ledit microgranule immédiat étant constitué soit d'un mélange d'excipients, de cisplatine et éventuellement d'autres principes actifs, soit d'un grain support neutre enrobé d'un mélange d'excipients, de cisplatine et éventuellement d'autres principes actifs.

On entend par microgranule immédiat, un microgranule dont les excipients de formulation n'agissent pas de manière significative sur la vitesse de libération ou de diffusion du principe actif.

L'agent d'enrobage permettant la libération contrôlée du cisplatine ou éventuellement d'autres principes actifs est, de préférence, constitué d'un ou plusieurs polymères d'enrobage pharmaceutiquement acceptables, choisis en particulier parmi les polymères cellulosiques ou parmi les copolymères d'acide méthacrylique, et préférentiellement, les poly (éthylacrylate, méthylméthacrylate) commercialisés sous la marque Eudragit NE 30D®.

Le revêtement contenant l'agent d'enrobage décrit précédemment est avantageusement constitué d'un seul polymère, ou éventuellement d'un mélange de polymères et/ou d'une succession de couches de polymères.

Eventuellement, il peut être associé au polymère de la couche d'enrobage assurant la libération contrôlée, différents additifs usuels en particulier : un agent lubrifiant et/ou un agent plastifiant et/ou un agent tensioactif.

L'agent lubrifiant peut être constitué d'un lubrifiant usuel pharmaceutiquement acceptable, en particulier le talc.

L'agent plastifiant est, de préférence, constitué d'un agent plastifiant pharmaceutiquement acceptable choisi parmi les esters aliphatiques tels que les esters d'acides citrique, phtalique, oxalique, et préférentiellement, le triéthylcitrate.

Le tensioactif peut être de type anionique, cationique, amphotère ou préférentiellement de type non ionique, en particulier le polysorbate 80 commercialisé sous la marque Montanox 80®.

Avantageusement, il peut être appliqué entre le microgranule immédiat et le revêtement contenant l'agent d'enrobage, une couche dite d'enrobage protecteur ou de prémontage ; cette couche intercalaire ayant pour rôle d'isoler le principe actif du polymère utilisé dans le revêtement d'enrobage décrit ci-dessus.

L'addition de chlorure de sodium au mélange d'excipients permet de renforcer la stabilité du principe actif cisplatine. Le mélange d'excipients comprend donc avantageusement du chlorure de sodium.

Les microgranules selon l'invention contiennent avantageusement une teneur en cisplatine comprise entre 25 et 350 mg/g, et préférentiellement entre 50 et 60 mg/g.

La présente invention concerne également le procédé de préparation des microgranules à libération contrôlée contenant du cisplatine selon l'invention.

Ledit procédé consiste en un montage du cisplatine sur des grains supports neutres par pulvérisation d'une suspension de montage contenant du cisplatine en milieu hydro-alcoolique, en milieu alcoolique, ou en milieu aqueux.

La suspension de montage est préférentiellement aqueuse et contient un agent stabilisant comme le chlorure de sodium, un ou plusieurs agents liants comme l'hydroxypropylméthylcellulose ou le polyéthylèneglycol. Eventuellement, il peut être ajouté à la suspension de montage, un agent tensioactif tel que décrit précédemment.

Les microgranules immédiats une fois enrobés par l'agent d'enrobage permettant la libération contrôlée du cisplatine peuvent être lubrifiés avec du talc.

Les solvants utilisés dans les étapes de préparation des microgranules de la présente invention peuvent être de nature aqueuse, alcoolique ou hydro-alcoolique. Préférentiellement, l'eau sera utilisée comme unique solvant lors du procédé de fabrication.

Les microgranules selon l'invention peuvent être obtenus par extrusion-sphéronisation en mélangeant en une seule étape le cisplatine, des agents liants et des agents stabilisants en milieu aqueux.

Les microgranules décrits dans la présente invention sont obtenus par utilisation de tout équipement adéquat pour la préparation et l'enrobage de microgranules, bien connu de l'homme du métier et en particulier les équipements de type turbine conventionnelle, turbine perforée, lit d'air fluidisé, extruseur et sphéronisateur.

La présente invention a également pour objet une préparation pharmaceutique contenant les microgranules de cisplatine à libération contrôlée selon l'invention, éventuellement obtenus selon le procédé décrit ci-dessus, en une quantité permettant d'obtenir une dose unitaire comprise entre 10 et 50 mg de cisplatine.

Ladite préparation pharmaceutique contient de préférence un mélange de microgranules de cisplatine à libération contrôlée et d'un agent anticancéreux ―par exemple le fluoro-uracil, le S1, l'association de la vinblastine avec la bléomycine, l'association de l'étoposide avec la bléomycine, ou le paclitaxel― comme produit de combinaison pour une utilisation simultanée, séparée ou étalée dans le temps en thérapie anticancéreuse.

La présente invention concerne enfin l'utilisation des microgranules selon l'invention pour fabriquer un médicament, à administrer par voie orale, destiné à être utilisé à faibles doses, notamment inférieures ou égales à environ 20 mg/m²/jour.

Ledit médicament peut être avantageusement utilisé en polychimiothérapie et/ou en association avec une radiothérapie, pour obtenir une concentration sanguine moyenne en cisplatine comprise entre 0,5 et 1,0 µg/ml.

Les exemples suivants illustrent l'invention sans en limiter la portée. Les pourcentages sont exprimés en masse sauf indication contraire.

### Exemple 1 : Protocole de préparation de microgranules immédiats par montage du cisplatine sur grains neutres.

### • Préparation de la suspension de montage.

- Pour une quantité de cisplatine de 100 g peser les excipients de montage dans les proportions adéquates,
- Placer le solvant ou le mélange de solvant dans un récipient sous agitation,
- Ajouter lentement le liant ou le mélange de liants, agiter jusqu'à obtenir une solution homogène,
- Ajouter le principe actif au moment du montage, agiter jusqu'à obtenir une suspension homogène.

### • Montage du principe actif sur les grains supports neutres

- Placer la quantité nécessaire de Neutres 20® (commercialisées par la société Np-pharm dont la granulométrie moyenne est comprise entre 0,7 et 0,9 mm, composées de 75 % de saccharose et de 25 % d'amidon de maïs) dans l'appareil retenu pour le montage du principe actif.
- Effectuer le montage du principe actif, sur les Neutres 20®, par pulvérisation continue de la suspension décrite ci-dessus,
- Tamiser la masse de microgranules obtenue,
- Sécher les microgranules en turbine à température ambiante.

### Exemple 2 : Préparation des microgranules immédiats A

Pour une quantité de cisplatine de 100g peser les excipients de montage suivants dans les proportions indiquées

| | | |
|---|---|---|
| PEG 4000 | 18g | 75% de l'extrait sec |
| PHARMACOAT 603® | 9g | 25% de l'extrait sec |
| EAU PURIFIEE | 10g | 5% du solvant |
| ALCOOL ETHYLIQUE 95% | 190g | 95% du solvant |

Formule finale :

| | |
|---|---|
| CISPLATINE | 22,3% |
| NEUTRES 20® | 72,4% |
| PEG 4000 | 4,0% |
| PHARMACOAT 603 ® | 1,3% |
| TENEUR THÉORIQUE | 223 mg/g |

### Exemple 3 : Préparation des microgranules immédiats B Pour une quantité de cisplatine de 100 g peser les excipients de montage suivants dans les proportions indiquées

| | | |
|---|---|---|
| PEG 4000 | 18,2 g | 30,5 % de l'extrait sec |
| PHARMACOAT 603® | 6,0 g | 10,1 % de l'extrait sec |
| CHLORURE DE SODIUM | 30,2 g | 50,7 % de l'extrait sec |
| MONTANOX 80® | 5,2 g | 8,7 % de l'extrait sec |
| EAU PURIFIÉE | 303,0 g | 100% du solvant |

Formule finale :

| | |
|---|---|
| CISPLATINE | 5,5% |
| NEUTRES 20® | 91,3% |
| PEG 4000 | 1,0% |
| PHARMACOAT 603® | 0,3% |
| MONTANOX 80® | 0,3% |
| CHLORURE DE SODIUM | 1,6% |
| TENEUR THÉORIQUE | 55 mg/g |

### Exemple 4 : Préparation des microgranules immédiats C

Pour une quantité de cisplatine de 100 g peser les excipients de montage suivants dans les proportions indiquées

| | | |
|---|---|---|
| PEG 4000 | 18,0 g | 28,0% de l'extrait sec |
| PHARMACOAT 603® | 6,0 g | 9,4% de l'extrait sec |
| CHLORURE DE SODIUM | 30,0 g | 46,7% de l'extrait sec |
| MONTANOX 80® | 10,2 g | 15,9% de l'extrait sec |
| EAU PURIFIÉE | 302,2 g | 100% du solvant |

Formule finale :

| | |
|---|---|
| CISPLATINE | 5,4% |
| NEUTRES 20® | 91,0% |
| PEG 4000 | 1,0% |
| PHARMACOAT 603® | 0,3% |
| MONTANOX 80® | 0,7% |
| CHLORURE DE SODIUM | 1,6% |
| TENEUR THÉORIQUE | 54 mg/g |

### Exemple 5 : Préparation des microgranules immédiats D

Pour une quantité de cisplatine de 100 g peser les excipients de montage suivants dans les proportions indiquées

| | | |
|---|---|---|
| PEG 4000 | 18,0 g | 28,0% de l'extrait sec |
| PHARMACOAT 603® | 6,0 g | 9,4% de l'extrait sec |
| CHLORURE DE SODIUM | 30,0 g | 46,7% de l'extrait sec |
| MONTANOX 80® | 10,2 g | 15,9% de l'extrait sec |
| EAU PURIFIÉE | 299,6 g | 100% du solvant |

Formule finale :

| | |
|---|---|
| CISPLATINE | 5,4% |
| NEUTRES 20® | 91,0% |
| PEG 4000 | 1,0% |
| PHARMACOAT 603® | 0,3% |
| MONTANOX 80® | 0,6% |
| CHLORURE DE SODIUM | 1,7% |
| TENEUR THÉORIQUE | 54 mg/g |

### Exemple 6 : Préparation des microgranules immédiats E

Pour une quantité de cisplatine de 100 g peser les excipients de montage suivants dans les proportions indiquées

| | | |
|---|---|---|
| PEG 4000 | 18 g | 72% de l'extrait sec |
| PHARMACOAT 603® | 6 g | 24% de l'extrait sec |
| CHLORURE DE SODIUM | 1 g | 4% de l'extrait sec |
| EAU PURIFIÉE | 240 g | 100% du solvant |

Formule finale :

| | |
|---|---|
| CISPLATINE | 5,5% |
| NEUTRES 20® | 93,1% |
| PEG 4000 | 1,0% |
| PHARMACOAT 603® | 0,3% |
| CHLORURE DE SODIUM | 0,1% |
| TENEUR THÉORIQUE | 55 mg/g |

### Exemple 7 : Protocole de préparation de microgranules à libération prolongée

### • Préparation de la suspension d'enrobage

- Peser les excipients d'enrobage dans les proportions adéquates,
- Placer le solvant ou le mélange de solvants dans un récipient sous agitation,
- Ajouter lentement l'agent d'enrobage ou le mélange d'agent d'enrobage, agiter jusqu'à obtenir une solution homogène.
- Ajouter lentement les différents additifs, agiter jusqu'à obtenir une suspension homogène,
- Maintenir l'agitation durant toute la phase d'enrobage.

### • Enrobage des microgranules

- Placer une fraction des microgranules immédiats obtenus selon le protocole de l'exemple 1 dans le matériel retenu pour l'enrobage,
- Effectuer l'enrobage des microgranules par pulvérisation continue de la suspension décrite ci-dessus,
- Tamiser la masse de microgranules obtenue,
- Sécher les microgranules à température ambiante,
- Répéter cette suite d'opérations le nombre de fois nécessaire à l'obtention de la cinétique désirée.

### Exemple 8 : Préparation des microgranules à libération prolongée A1

Peser les excipients d'enrobage suivants dans les proportions indiquées

| | | |
|---|---|---|
| EUDRAGIT L 30 D® | 10,0g | Extrait sec d'Eudragit® = 30% de la masse d'Eudragit® pesée |
| TRIETHYL CITRATE | 0,3g | Extrait sec de TRIETHYL CITRATE = 10% d'extrait sec d'Eudragit® |
| EAU PURIFIEE | 5,0g | Solvant de dilution = 50% de la masse d'Eudragit® pesée |

Enrober les microgranules immédiats A de l'exemple 2
Formule finale :

| | |
|---|---|
| CISPLATINE | 18,2% |
| NEUTRES 20® | 59,1% |
| PEG 4000 | 3,3% |
| PHARMACOAT 603® | 1,1% |
| EUDRAGIT L 100-55® | 0,8% |
| EUDRAGIT L 30 D® | 10,2% |
| EUDRAGIT NE 3 0 D® | 1,0% |
| TRIETHYL CITRATE | 1,0% |
| TALC | 5,3% |
| TENEUR THÉORIQUE | 182 mg/g |

### Exemple 9 : Préparation des microgranules à libération prolongée A2

Peser les excipients d'enrobage suivants dans les proportions indiquées

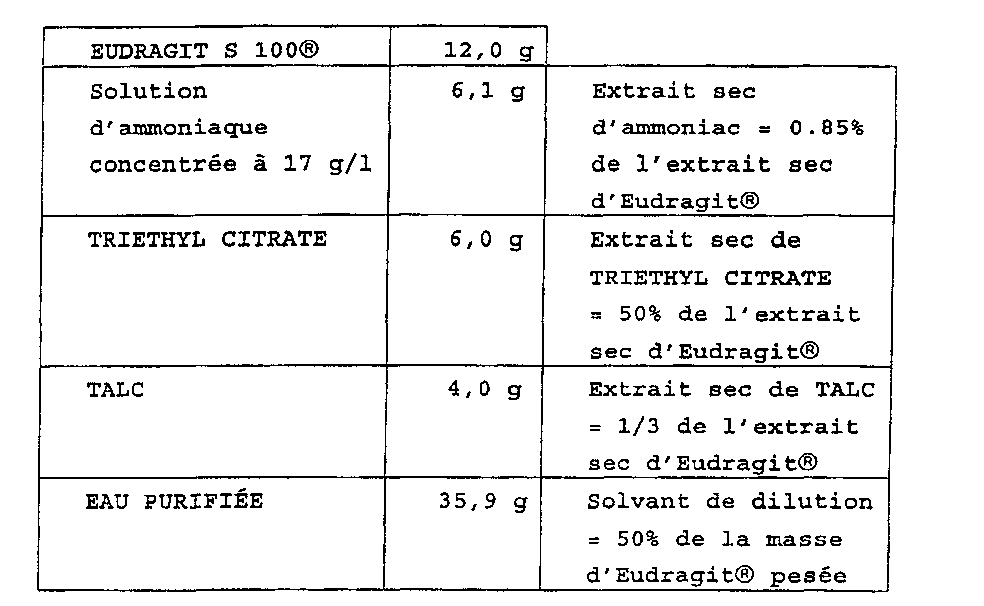

Enrober les microgranules immédiats A de l'exemple 2
Formule finale :

| | |
|---|---|
| CISPLATINE | 20,7% |
| NEUTRES 20® | 67,2% |
| PEG 4000 | 3,8% |
| PHARMACOAT 603® | 1,2% |
| EUDRAGIT S 100® | 3,77% |
| AMMONIAC | 0,03% |
| TRIETHYL CITRATE | 1,4% |
| TALC | 1,9% |
| TENEUR THÉORIQUE | 207 mg/g |

### Exemple 10 : Préparation des microgranules à libération prolongée E1

Peser les excipients d'enrobage suivants dans les proportions indiquées

| | | |
|---|---|---|
| EUDRAGIT L 30 D® | 10,0 g | Extrait sec d'Eudragit® = 30% de la masse d'Eudragit® pesée |
| TRIETHYL CITRATE | 0,3 g | Extrait sec de TRIETHYL CITRATE = 10% de l'extrait sec d'Eudragit® |
| EAU PURIFIÉE | 5,0 g | Solvant de dilution = 50% de la masse d'Eudragit® pesée |

Enrober les microgranules immédiats E de l'exemple 6
Formule finale :

| | |
|---|---|
| CISPLATINE | 4,5% |
| NEUTRES 20® | 75,35% |
| PEG 4000 | 0,8% |
| PHARMACOAT 603® | 0,3% |
| CHLORURE DE SODIUM | 0,05% |
| EUDRAGIT L 30 ® | 9,0% |
| TRIETHYL CITRATE | 0,9% |
| TALC | 9,1% |
| TENEUR THÉORIQUE | 45 mg/g |

### Exemple 11 : Préparation des microgranules à libération prolongée E2

Peser les excipients d'enrobage suivants dans les proportions indiquées

| | | |
|---|---|---|
| EUDRAGIT NE 30 D® | 10,0 g | Extrait sec d'Eudragit® = 30% de la masse d'Eudragit® pesée |
| EAU PURIFIÉE | 5,0 g | Solvant de dilution = 50% de la masse d'Eudragit® pesée |

Enrober les microgranules immédiats E de l'exemple 6
Formule finale :

| | |
|---|---|
| CISPLATINE | 5,4% |
| NEUTRES 20® | 91,3% |
| PEG 4000 | 0,95% |
| PHARMACOAT 603® | 0,3% |
| CHLORURE DE SODIUM | 0,05% |
| EUDRAGIT NE 30 D® | 1,0% |
| TALC | 1,0% |
| TENEUR THÉORIQUE | 54 mg/g |

### Exemple 12 : Préparation des microgranules à libération prolongée E3

Peser les excipients d'enrobage suivants dans les proportions indiquées

| | | |
|---|---|---|
| EUDRAGIT NE 30 D® | 10,0 g | Extrait sec d'Eudragit® = 30% de la masse d'Eudragit® pesée |
| EAU PURIFIÉE | 5,0 g | Solvant de dilution = 50% de la masse d'Eudragit® pesée |

Enrober les microgranules immédiats E de l'exemple 6
Formule finale :

| | |
|---|---|
| CISPLATINE | 5,3% |
| NEUTRES 20® | 89,6% |
| PEG 4000 | 0,95% |
| PHARMACOAT ® | 0,3% |
| CHLORURE DE SODIUM | 0,05% |
| EUDRAGIT NE 30 D® | 1,9% |
| TALC | 1,9% |
| TENEUR THÉORIQUE | 53 mg/g |

### Exemple 13 : Préparation des microgranules à libération prolongée E4

Peser les excipients d'enrobage suivants dans les proportions indiquées

| | | |
|---|---|---|
| EUDRAGIT NE 30 D® | 10,0 g | Extrait sec d'Eudragit® = 30% de la masse d'Eudragit® pesée |
| TALC | 3,0 g | Extrait sec de TALC = 100% de l'extrait sec d'Eudragit® |
| EAU PURIFIEE | 5,0 g | Solvant de dilution = 50% de la masse d'Eudragit® pesée |

Peser les excipients d'enrobage suivants dans les proportions indiquées

| | | |
|---|---|---|
| EUDRAGIT L 30 D® | 10,0 g | Extrait sec d'Eudragit® = 30% de la masse d'Eudragit® pesée |
| TALC | 3,0 g | Extrait sec de TALC = 100% de l'extrait sec d'Eudragit® |
| TRIETHYL CITRATE | 0,3 g | Extrait sec de TRIETHYL CITRATE = 10% de l'extrait sec d'Eudragit® |
| EAU PURIFIEE | 5,0 g | Solvant de dilution = 50% de la masse d'Eudragit® pesée |

Enrober les microgranules immédiats E de l'exemple 6
Formule finale :

| | |
|---|---|
| CISPLATINE | 4,4 % |
| NEUTRES 20® | 74,8 % |
| PEG 4000 | 0,8% |
| PHARMACOAT 603® | 0,3% |
| CHLORURE DE SODIUM | 0,04% |
| EUDRAGIT NE 30 D® | 0,8% |
| EUDRAGIT L 30 D@ | 8,56% |
| TRIETHYL CITRATE | 0,9% |
| TALC | 9,4% |
| TENEUR THÉORIQUE | 44 mg/g |

### Etudes précliniques et cliniques

### • Biodisponibilité chez l'animal

La biodisponibilité du cisplatine microgranules est comparée soit à celle du cisplatine administré classiquement par voie I.V. (biodisponibilité absolue) soit à celle de la formulation injectable administrée par voie orale (biodisponibilité relative) chez le chien, le rat et le singe après administration unique ou répétée (1 administration quotidienne pendant 7 jours).

Des prises de sang sont effectuées 15 min, 30 min, 1,2,4,8 et 24 heures après l'administration orale ou I.V. du cisplatine. La concentration plasmatique en platine total est déterminée par absorption atomique spectrophotométrique.

Les tableaux 1 et 2 ci-dessous regroupent la moyenne des résultats obtenus en ce qui concerne la biodisponibilité absolue et la biodisponibilité relative.

**Tableau 1 :**

| BIODISPONIBILITE ABSOLUE | | | | | |
|---|---|---|---|---|---|
| | A | A1 | CDDP IV administré per-os | E2 | E3 |
| Chien-n=3 1mg/kg (20 mg/m²) | 63,4% | 46,0% | 47,3% | 66,1% | 49,0% |
| Singe-n=3 1mg/kg | 17,3% | 11,4% | 8,0 % | 13,7% | 7,7% |
| Rat-n=3 1mg/kg ( 6 mg/m²) | 47,7% | 41,9% | 44,2% | | |

**Tableau 2 :**

| BIODISPONIBILITE RELATIVE PAR RAPPORT A LA FORMULATION INJECTABLE ADMINISTREE PER OS CHEZ LE RAT | | | | | |
|---|---|---|---|---|---|
| | E | E1 | E2 | E3 | E4 |
| Administration unique 2mg/kg (12 mg/m²) n=4 | 117% | 118% | 138% | 69% | 163% |
| Administration répétée 0,5mg/kg/jour (3 mg/m²/jour) pendant 7 jours n=5 | 173% | 249% | 206% | 164% | 128% |

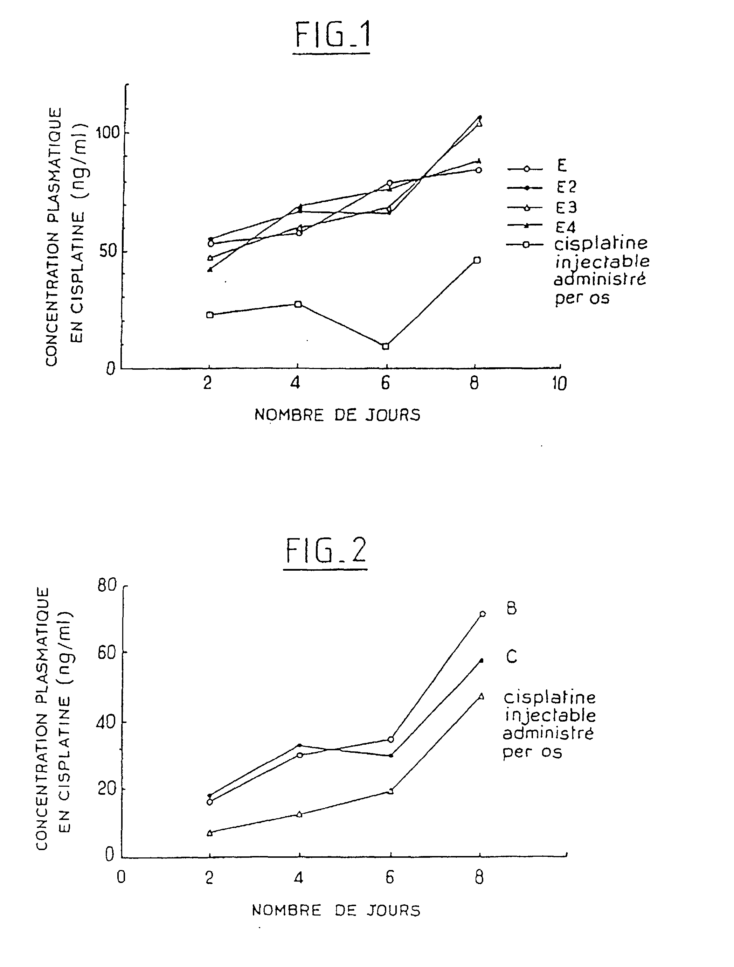

Par ailleurs, les concentrations plasmatiques résiduelles en cisplatine sont mesurées chez le rat après administration des formulations décrites dans la présente invention comparativement à la formulation injectable administrée per os à raison de 2 mg/kg/jour (12 mg/m²/jour) pendant 7 jours consécutifs en une prise unique journalière. Les résultats sont illustrés par les courbes présentées en annexe figures 1 et 2.

La figure 1 représente la concentration plasmatique en cisplatine en fonction du temps de cinq groupes de cinq rats auxquels on a respectivement administré 2 mg/kg/jour (12 mg/m²/jour) de microgranules E, E2, E3, E4 par voie orale et du cisplatine injectable administré per os, pendant 7 jours consécutifs à raison d'une prise unique par jour. La concentration plasmatique en cisplatine est mesurée 24 heures après chaque administration.

La figure 2 représente la concentration plasmatique en cisplatine en fonction du temps de trois groupes de cinq rats auxquels on a respectivement administré des microgranules B et C par voie orale et du cisplatine injectable administré per os dans les mêmes conditions que celles de la figure 1.

L'ensemble de ces résultats montre que :
- l'absorption digestive des formulations orales cisplatine microgranules selon l'invention est supérieure à celle de la formulation injectable administrée per os chez l'ensemble des espèces testées, que ce soit après administration unique ou répétée,
- les concentrations plasmatiques en platine mesurées après administration du cisplatine microgranules selon l'invention sont plus prolongées que celles obtenues après administration orale d'une dose équivalente d'une formulation injectable.

### • Toxicologie aiguë et histopathologie chez le chien de race Beagle traité par des microgranules E2

L'objet de cette étude est de déterminer la dose maximale tolérée (DMT) de cisplatine microgranules E2 chez le chien après l'administration d'une dose unique.
Un total de 6 chiens mâles et 6 chiens femelles est utilisé. Des doses de 0,2, 0,5, 1,0 et 2,0 mg/kg (4, 10, 20 et 40 mg/m²) de cisplatine microgranules par voie orale et une dose de 1 mg/kg (20 mg/m²) de cisplatine de marque Cisplatyl® par voie intraveineuse sont administrées aux animaux.
La concentration plasmatique en platine est mesurée 30 min, 1, 4 et 24 heures après l'administration. L'examen histopathologique est réalisé à la fin du 14ème jour.
Les animaux auxquels on a administré 1 ou 2 mg/kg (20 ou 40 mg/m²) de cisplatine microgranules et 1 mg/kg de cisplatine I.V. ont souffert de vomissements.
On a noté des cas de diarrhées chez les animaux traités avec 1 mg/kg de cisplatine I.V. et 2 mg/kg de cisplatine microgranules. La dose orale de cisplatine microgranules 2 mg/kg a également provoqué une perte de poids, une diminution de la prise de nourriture, une diminution de poids de la rate et du thymus, un ulcère duodénal chez un chien et de légères lésions rénales.
L'examen histopathologique montre que presque tous les animaux traités par 1,0 mg/kg et 2,0 mg/kg de cisplatine microgranules souffrent d'hypoplasie de la moelle osseuse modérée fonction de la dose de cisplatine administrée.
La dose orale de cisplatine microgranules 1 mg/kg induit des effets toxiques moindres que la dose de cisplatine I.V. 1 mg/kg. Par ailleurs, ces effets toxiques ne sont pas irréversibles.
Les doses plus faibles de cisplatine microgranules administrées par voie orale sont particulièrement bien tolérées et on ne remarque que des changements de peu d'importance dans l'intestin.
La dose maximale tolérée (DMT) de cisplatine microgranules E2 chez le chien après l'administration d'une dose unique se situe donc entre 1,0 mg/kg et 2,0 mg/kg.
La biodisponibilité absolue de platine se situe entre 41 et 77% (moyenne 64%). De plus, la variabilité inter animaux est très faible en ce qui concerne les concentrations plasmatiques maximales.

### • Toxicologie subaiguë chez le chien de race Beagle après administration de doses répétées de cisplatine microgranules E2

Des doses de zéro (gélules vides), 0,25 et 1,0 mg/kg/jour de cisplatine microgranules sont administrées à des chiens mâles (3 par groupe) sur une période de 4 semaines.
Le poids des chiens est contrôlé deux fois par semaine, la prise de nourriture et d'eau est vérifiée une fois par semaine. Des examens biochimiques et hématologiques du sang ainsi que des urines sont pratiqués avant et après l'expérimentation. Les concentrations sanguines en platine sont mesurées une fois par semaine par absorption atomique spectrométrique.

L'examen histopathologique et macroscopique est réalisé à la fin de l'expérimentation.

Dans le groupe de 1,0 mg/kg (20 mg/m²):
L'autopsie montre des lésions hémorragiques du pylore, du duodénum et de la région iléo-caecale du colon. L'examen histopathologique montre que presque tous les animaux traités par 1,0 mg/kg de cisplatine microgranules souffrent d'hypoplasie de la moelle osseuse prononcée, d'érosion dans la région gastro-intestinale, de dégénération granulaire des tubules rénaux et de lésions nécrotiques des testicules.

Dans le groupe de 0,25 mg/kg (5 mg/m²) :
La tolérance générale est satisfaisante pendant toute la durée du traitement, avec une légère réduction de prise de nourriture. L'examen histopathologique révèle une légère hypoplasie de la moelle osseuse.

### • Biodisponibilité absolue chez l'homme des microgranules E2

Huit malades ayant un cancer d'une nature sensible au cisplatine reçoivent en fonction de la randomisation une dose unique de 10 mg/m² de cisplatine soit oralement sous forme de microgranules E2 (gélules à 2,5 et 5 mg) soit en perfusion intraveineuse de 30 minutes (CISPLATYL® BELLON).
Les taux plasmatiques de platine total sont mesurés avant l'administration des médicaments, puis 15 min, 30 min, 45 min, 1h, 1h30, 2h, 4h, 8h, 12h et 24h après l'administration des médicaments.
Huit malades ont reçu les microgranules E2 et huit autres malades ont reçu le CISPLATYL® BELLON par voie intraveineuse. La valeur moyenne de la biodisponibilité absolue de cisplatine microgranules E2 est de 39 %. La variabilité interindividuelle après administration de microgranules E2 est modérée et similaire à celle observée après administration de CISPLATYL® I.V. (CV % = 27,4 % p.o. et 23,7 % I.V. respectivement.

## Revendications

1. Microgranules à libération contrôlée, pour administration orale, contenant du cisplatine, dont la granulométrie moyenne est comprise entre 0,4 et 1,5 mm, notamment entre 1 et 1,25 mm.

2. Microgranules selon la revendication 1, **caractérisés en ce que** chaque microgranule comprend un microgranule immédiat sur lequel est monté un revêtement contenant un agent d'enrobage permettant la libération contrôlée du cisplatine et/ou d'autres principes actifs, ledit microgranule immédiat étant constitué soit d'un mélange d'excipients, de cisplatine et éventuellement d'autres principes actifs, soit d'un grain support neutre enrobé d'un mélange d'excipients, de cisplatine et éventuellement d'autres principes actifs.

3. Microgranules selon la revendication 2, **caractérisés en ce que** l'agent d'enrobage permettant la libération contrôlée du cisplatine ou éventuellement d'autres principes actifs est choisi parmi les polymères cellulosiques ou parmi les copolymères d'acide méthacrylique.

4. Microgranules selon la revendication 3, **caractérisés en ce que** l'agent d'enrobage est choisi parmi les poly(éthylacrylate, méthylméthacrylate).

5. Microgranules selon l'une des revendications 2 à 4, **caractérisés en ce que** le revêtement contenant l'agent d'enrobage est constitué d'un seul polymère, ou d'un mélange de polymères et/ou d'une succession de couches de polymères.

6. Microgranules selon l'une des revendications 2 à 5, **caractérisés en ce que** l'on associe à l'agent d'enrobage un agent lubrifiant, comme le talc, et/ou un agent plastifiant, comme le triéthylcitrate, et/ou un agent tensioactif, comme le polysorbate 80.

7. Microgranules selon l'une des revendications 2 à 6, **caractérisés en ce qu'**une couche d'enrobage protecteur est montée entre le microgranule immédiat et le revêtement contenant l'agent d'enrobage.

8. Microgranules selon l'une des revendications 2 à 7, **caractérisés en ce que** le mélange d'excipients comprend du chlorure de sodium.

9. Microgranules selon l'une des revendications 2 à 8, **caractérisés en ce que** leur teneur en cisplatine est comprise entre 25 et 350 mg/g.

10. Microgranules selon la revendication 9, **caractérisés en ce que** leur teneur en cisplatine est comprise entre 50 et 60 mg/g.

11. Procédé de préparation de microgranules selon l'une des revendications 2 à 10, **caractérisé en ce qu'**un montage du cisplatine sur des grains supports neutres est réalisé par pulvérisation d'une suspension de montage contenant du cisplatine en milieu hydro-alcoolique, en milieu alcoolique, ou en milieu aqueux.

12. Procédé de préparation de microgranules selon la revendication 11, **caractérisé en ce que** la suspension de montage est aqueuse et contient un agent stabilisant comme le chlorure de sodium, un ou plusieurs agents liants comme l'hydroxypropylméthylcellulose ou le polyéthylèneglycol, et/ou un agent tensioactif.

13. Procédé de préparation de microgranules selon la revendication 11 ou 12, **caractérisé en ce que** les microgranules immédiats enrobés par l'agent d'enrobage sont lubrifiés avec du talc.

14. Préparation pharmaceutique, **caractérisée en ce qu'**elle contient des microgranules selon l'une des revendications 1 à 10 ou obtenus par le procédé selon l'une des revendications 11 à 13, en une quantité permettant d'obtenir une dose unitaire comprise entre 10 et 50 mg de cisplatine.

15. Préparation pharmaceutique selon la revendication 14, **caractérisée en ce qu'**elle contient un mélange de microgranules de cisplatine et d'un agent anticancéreux, comme produit de combinaison pour une utilisation simultanée, séparée ou étalée dans le temps en thérapie anticancéreuse.

16. Préparation pharmaceutique selon la revendication 15, **caractérisée en ce que** l'agent anticancéreux est le fluoro-uracil, le S1, (un mélange tegafur /5-chloro-2,4-dihydroxpyridine/ acide oxonique dans une proportion molaire 1/0,4/1) l'association de la vinblastine avec la bléomycine, l'association de l'étoposide avec la bléomycine ou le paclitaxel.

17. Utilisation des microgranules selon les revendications 1 à 10 ou obtenus par le procédé selon les revendications 11 à 13, pour fabriquer un médicament, à administrer par voie orale, destiné à être utilisé à faibles doses, notamment inférieures ou égales à environ 20 mg/m²/jour.

18. Utilisation selon la revendication 17, **caractérisée en ce que** le médicament est utilisé en polychimiothérapie et/ou en association avec une radiothérapie.

19. Utilisation selon la revendication 17 ou la revendication 18, pour obtenir une concentration sanguine moyenne en cisplatine comprise entre 0,5 et 1,0 µg/ml.

## Patentansprüche

1. Cisplatin enthaltende Mikrokörnchen mit kontrollierter Freisetzung für eine orale Verabreichung, deren mittlere Korngröße (Granulometrie) zwischen 0,4 und 1,5 mm, insbesondere zwischen 1 und 1,25 mm beträgt.

2. Mikrokörnchen nach Anspruch 1, **dadurch gekennzeichnet, dass** jedes Mikrokörnchen ein Mikrokörnchen mit unmittelbarer Freisetzung ("microgranule immédiat") umfasst, auf welches ein Überzug aufgebracht ist, welcher ein Umhüllungsmittel, welches die kontrollierte Freisetzung des Cisplatin und/oder von anderen Wirkstoffen erlaubt, enthält, wobei das Mikrokörnchen mit unmittelbarer Freisetzung entweder aus einer Mischung von Trägersubstanzen, Cisplatin und gegebenenfalls anderen Wirkstoffen oder aus einem neutralen Trägerkorn, welches von einer Mischung von Trägersubstanzen, Cisplatin und gegebenenfalls anderen Wirkstoffen umhüllt wird, gebildet wird.

3. Mikrokörnchen nach Anspruch 2, **dadurch gekennzeichnet, dass** das Umhüllungsmittel, welches die kontrollierte Freisetzung des Cisplatin oder gegebenenfalls von anderen Wirkstoffen erlaubt, unter den cellulosischen Polymeren oder unter den Copolymeren von Methacrylsäure ausgewählt wird.

4. Mikrokörnchen nach Anspruch 3, **dadurch gekennzeichnet, dass** das Umhüllungsmittel unter den Poly(ethylacrylat, methyl methacrylat)-Polymeren ausgewählt wird.

5. Mikrokörnchen nach einem der Ansprüche 2 bis 4, **dadurch gekennzeichnet, dass** der Überzug, welcher das Umhüllungsmittel enthält, aus einem einzigen Polymer oder einer Mischung von Polymeren und/oder einer Aufeinanderfolge von Schichten von Polymeren gebildet wird.

6. Mikrokörnchen nach einem der Ansprüche 2 bis 5, **dadurch gekennzeichnet, dass** man mit dem Umhüllungsmittel ein Gleitmittel, wie Talkum, und/oder einen Weichmacher, wie Triethylcitrat, und/oder ein grenzflächenaktives Mittel, wie Polysorbat 80, kombiniert.

7. Mikrokörnchen nach einem der Ansprüche 2 bis 6, **dadurch gekennzeichnet, dass** eine schützende Umhüllungsschicht zwischen dem Mikrokörnchen mit unmittelbarer Freisetzung und dem Überzug, welcher das Umhüllungsmittel enthält, aufgebracht wird.

8. Mikrokörnchen nach einem der Ansprüche 2 bis 7, **dadurch gekennzeichnet, dass** die Mischung von Trägersubstanzen Natriumchlorid umfasst.

9. Mikrokörnchen nach einem der Ansprüche 2 bis 8, **dadurch gekennzeichnet, dass** deren Gehalt an Cisplatin zwischen 25 und 350 mg/g beträgt.

10. Mikrokörnchen nach Anspruch 9, **dadurch gekennzeichnet, dass** deren Gehalt an Cisplatin zwischen 50 und 60 mg/g beträgt.

11. Verfahren zur Herstellung von Mikrokörnchen nach einem der Ansprüche 2 bis 10, **dadurch gekennzeichnet, dass** ein Aufbringen des Cisplatins auf neutrale Trägerkörner durch Zerstäubung einer zum Aufbringen dienenden Suspension, welche Cisplatin in einem wässrig-alkoholischen Medium, in einem alkoholischen Medium oder in einem wässrigen Medium enthält, ausgeführt wird.

12. Verfahren zur Herstellung von Mikrokörnchen nach Anspruch 11, **dadurch gekennzeichnet, dass** die zum Aufbringen dienende Suspension wässrig ist und ein Stabilisierungsmittel, wie Natriumchlorid, ein oder mehrere Bindemittel, wie Hydroxypropylmethylcellulose oder Polyethylenglycol, und/oder ein grenzflächenaktives Mittel enthält.

13. Verfahren zur Herstellung von Mikrokörnchen nach Anspruch 11 oder 12, **dadurch gekennzeichnet, dass** die durch das Umhüllungsmittel überzogenen Mikrokörnchen mit unmittelbarer Freisetzung mit Talkum gleitfähig gemacht werden.

14. Pharmazeutische Zubereitung, **dadurch gekennzeichnet, dass** sie Mikrokörnchen nach einem der Ansprüche 1 bis 10 oder erhalten durch das Verfahren nach einem der Ansprüche 11 bis 13 in einer Menge, welche erlaubt, eine Einheitsdosis zwischen 10 und 50 mg Cisplatin zu erhalten, enthält.

15. Pharmazeutische Zubereitung nach Anspruch 14, **dadurch gekennzeichnet, dass** sie eine Mischung von Mikrokörnchen von Cisplatin und einem Antikrebsmittel enthält, als Kombinationsprodukt für eine gleichzeitige, getrennte oder zeitlich gestaffelte Verwendung in der Antikrebstherapie.

16. Pharmazeutische Zubereitung nach Anspruch 15, **dadurch gekennzeichnet, dass** das Antikrebsmittel Fluoruracil, S1 (eine Mischung von Tegafur/5-Chlor-2,4-dihydroxypyridin/Oxonsäure in einem Molverhältnis von 1/0,4/1), die Kombination von Vinblastin mit Bleomycin, die Kombination von Etoposid mit Bleomycin oder Paclitaxel ist.

17. Verwendung der Mikrokörnchen nach den Ansprüchen 1 bis 10 oder erhalten durch das Verfahren nach den Ansprüchen 11 bis 13 zur Herstellung eines auf oralem Wege zu verabreichenden Arzneimittels, welches dazu bestimmt ist, in niedrigen Dosen, insbesondere unter oder gleich ungefähr 20 mg/m²/Tag, eingesetzt zu werden.

18. Verwendung nach Anspruch 17, **dadurch gekennzeichnet, dass** das Arzneimittel in einer Polychemotherapie und/oder in Kombination mit einer Strahlentherapie eingesetzt wird.

19. Verwendung nach Anspruch 17 oder Anspruch 18 um eine mittlere Konzentration von Cisplatin im Blut zwischen 0,5 und 1,0 µg/ml zu erhalten.

## Claims

1. Controlled-release microgranules for oral administration containing cisplatin, the mean particle size of which is between 0.4 and 1.5 mm, in particular between 1 and 1.25 mm.

2. Microgranules according to Claim 1, **characterized in that** each microgranule comprises an immediate microgranule to which is fixed a coating containing a coating agent which makes possible the controlled release of cisplatin and/or of other active principles, the said immediate microgranule being composed either of a mixture of excipients, of cisplatin and optionally of other active principles or of a neutral support grain coated with a mixture of excipients, of cisplatin and optionally of other active principles.

3. Microgranules according to Claim 2, **characterized in that** the coating agent which makes possible the controlled release of cisplatin or optionally of other active principles is chosen from cellulose polymers or from methacrylic acid copolymers.

4. Microgranules according to Claim 3, **characterized in that** the coating agent is chosen from poly(ethyl acrylate, methyl methacrylate)s.

5. Microgranules according to one of Claims 2 to 4, **characterized in that** the coating containing the coating agent is composed of a single polymer or of a mixture of polymers and/or of a sequence of polymer layers.

6. Microgranules according to one of Claims 2 to 5,
**characterized in that** the coating agent is combined with a lubricating agent, such as talc, and/or a plasticizing agent, such as triethyl citrate, and/or a surface-active agent, such as polysorbate 80.

7. Microgranules according to one of Claims 2 to 6, **characterized in that** a protective coating layer is fixed between the immediate microgranule and the coating containing the coating agent.

8. Microgranules according to one of Claims 2 to 7, **characterized in that** the mixture of excipients comprises sodium chloride.

9. Microgranules according to one of Claims 2 to 8, **characterized in that** their cisplatin content is between 25 and 350 mg/g.

10. Microgranules according to Claim 9, **characterized in that** their cisplatin content is between 50 and 60 mg/g.

11. Process for the preparation of microgranules according to one of Claims 2 to 10, **characterized in that** fixing cisplatin to neutral support grains is carried out by spraying a fixing suspension containing cisplatin in aqueous/alcoholic medium, in alcoholic medium or in aqueous medium.

12. Process for the preparation of microgranules according to Claim 11, **characterized in that** the fixing suspension is aqueous and contains a stabilizing agent, such as sodium chloride, one or more binding agents, such as hydroxypropylmethylcellulose or polyethylene glycol, and a surface-active agent.

13. Process for the preparation of microgranules according to Claim 11 or 12, **characterized in that** the immediate microgranules coated with the coating agent are lubricated with talc.

14. Pharmaceutical preparation, **characterized in that** it contains microgranules according to one of Claims 1 to 10 or obtained by the process according to one of Claims 11 to 13 in an amount which makes it possible to obtain a unit dose of between 10 and 50 mg of cisplatin.

15. Pharmaceutical preparation according to Claim 14, **characterized in that** it contains a mixture of microgranules of cisplatin and of an anticancer agent as combination product for a use in anticancer therapy which is simultaneous, separate or spread out over time.

16. Pharmaceutical preparation according to Claim 15, **characterized in that** the anticancer agent is fluorouracil, S1 (a tegafur/5-chloro-2,4-dihydroxy-pyridine/oxonic acid mixture in a 1/0.4/1 molar proportion), the combination of vinblastine with bleomycin, the combination of etoposide with bleomycin, or paclitaxel.

17. Use of the microgranules according to Claims 1 to 10 or obtained by the process according to Claims 11 to 13 in manufacturing a medicament, to be administered by the oral route, intended to be used at low doses, in particular less than or equal to approximately 20 mg/m²/day.

18. Use according to Claim 17, **characterized in that** the medicament is used in polychemotherapy and/or in combination with a radiotherapy.

19. Use according to Claim 17 or Claim 18 in order to obtain an average cisplatin blood concentration of between 0.5 and 1.0 µg/ml.
